# EUROPÄISCHE PATENTSCHRIFT

(11) **EP 0 863 145 B1**
(45) Veröffentlichungstag und Bekanntmachung des Hinweises auf die Patenterteilung: **01.10.2003**
(21) Anmeldenummer: 98103244.4
(22) Anmeldetag: 25.02.1998
(51) Int. Cl.: C07F 7/08, C07F 7/18, A61K 7/42

(54) **Lichtschutzmittel**
Sunscreen compositions
Filtres solaires

(30) Priorität: 03.03.1997 EP 97103434
(43) Veröffentlichungstag der Anmeldung: 09.09.1998
(73) Patentinhaber: F. HOFFMANN-LA ROCHE AG, 4070 Basel (CH)
(72) Erfinder: Huber, Ulrich, 8703 Erlenbach (CH)
(74) Vertreter: Lederer, Franz, Dr.

(56) Entgegenhaltungen:
- CH-A- 484 695

## Beschreibung

Sonnenlicht beschleunigt die Alterung der Haut und ruft sogar Hautkrebs hervor, und zwar werden diese unerwünschten Effekte neben der UV-B-Strahlung vor allem durch die die Haut direkt bräunende UV-A-Strahlung mit Wellenlängen im Bereiche von etwa 320 bis 400 nm verursacht.

Bereits aus der Schweizer Patentschrift 484 695 ist bekannt, dass symmetrische Tris-(2',4'-dihydroxy phenyl)-1-3-5-triazine sehr effiziente UV-A/B-Lichtschutzmittel zum Schutz von Textilien sind. Ein wesentlicher Nachteil ist jedoch die ausgesprochen schlechte Löslichkeit dieser Substanzklasse in praktisch allen Lösungsmitteln, die eine Anwendung z.B. für kosmetische Applikationen nicht zulässt.

In der USP 3,896,125 sind o-Hydroxyphenyl-s-triazine beschrieben, die zwar UV-Licht absorbieren, aber unlöslich oder nur schwer löslich in nicht polaren Polymeren wie Polyethylen, Polypropylen oder Polyesterharzen sind.

Ferner sind aus der EP-A1-502 821 2-Hydroxyphenyl-3-triazine mit einem siliziumhaltigen Rest in 4-Stellung der 2-Hydroxyphenylgruppe zur Lichtstabilisierung von organischen Polymeren bekannt. In Formkörpern aus damit stabilisierten Polymeren verhindert der in Aussenschichten des Formkörpers enthaltene UV-Absorber das Eindringen von kurzwelligem, für Polymere besonders schädlichen Licht, d.h. vor allem von UV-B-Strahlung, in das Innere des Formkörpers.

Es besteht jedoch das dringende Bedürfnis, insbesondere von den Herstellern von Körperpflegeprodukten, die Verbindungsklasse der Hydroxyphenyl-s-triazine aufgrund ihrer Lichtschutzwirkung auch für andere Zwecke zugänglich zu machen. Dazu ist zumindest eine gute Löslichkeit in organischen Lösungsmitteln, insbesondere in kosmetischen Lösungsmitteln, und eine gute Photostabilität gefordert.

Es wurde nun gefunden, dass symmetrische Verbindungen der Formel I, 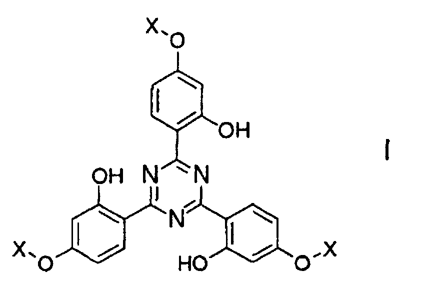 worin alle X über Spacergruppen Z an O gebundene Siliziumreste S sind oder zwei X über Spacergruppen Z an O gebundene Siliziumreste S sind und das dritte X H oder ein an O gebundener Siliziumrest S ist, wobei
die Spacergruppe Z eine gesättigte, einfach oder mehrfach ungesättigte Alkylgruppe mit 3 bis 12 C-Atomen und
der Siliziumrest S 1-8 Si-Atome enthält und aus 3-fach substituierten Alkyl-silylgruppen, Alkoxy-silylgruppen mit je 3-15 C-Atomen oder einem Oligosiloxan besteht,
gut in organischen und insbesondere in kosmetischen Lösungsmitteln löslich gemacht werden können und vorzügliche UV-A-Filter darstellen, indem sie bei ausgezeichneter Hautverträglichkeit und Stabilität (Licht, Hitze, Feuchtigkeit) eine starke Reduktion der Stresswirkung auf die Haut und damit eine Verzögerung der Hautalterung bewirken, d.h. Substanzen sind, welche die erythemerzeugende UV-A-Strahlung im Bereich von etwa 320 bis 400 nm absorbieren. Insbesondere weisen diese UV-A-Filter aber auch eine ausgezeichnete Photostabilität auf. Damit können sie in kosmetischen Emulsionen eingesetzt werden und ergeben leicht zu verarbeitende Präparate, die sich für Anwendungen als kosmetische Sonnenschutzmittel auf der Haut oder in Haarpflegemittel ausgezeichnet eignen.

Bevorzugt sind die Alkylgruppen der Spacergruppen Z in Stellung 2, 3, 4, 5 oder 6 mit den Siziliumresten S verbunden, weil Produkte, bei denen die Alkylgruppen der Spacergruppen Z in Stellung 2 oder 3 mit den Siliziumresten S verbunden sind, kostengünstiger sind und Produkte mit den Stellungen 4, 5 oder 6 in grösserer Reinheit hergestellt werden können.

Beispiele von Spacergruppen Z sind 3-Propyl-, 2-Propyl-, 3-Propenyl-, 2-Propen-3-yl-, 2-Propen-2-yl-, 2-Methyl-2-propyl, 2-Methyl-3-propenyl, 3-Butyl-, 2-Butyl-, 3-Butenyl-, 3-Methyl-2-butyl-, 2,4-Pentadien-5-yl-, 4-Pentyl-, 5-Pentyl-, 5-Hexyl-, 6-Hexyl- und 2-Dodecen-12-yl-.

Beispiele von Siliziumresten S sind -Si Me₃, Si Et₃, -Si Isopropenyl₃-, -Si tert.Butyl₃, Si sec.Butyl₃, -Si Me₂ tert. Butyl, -Si Me₂ Thexyl (Thexyl is 1,1,2-trimethylpropyl), -Si(OMe)₃, -Si(OEt)₃, -Si(OPh)₃ oder Oligosiloxan.

Ein geeignetes Oligosiloxan kann zum Beispiel sein -SiMeₘ(OSiMe₃)ₙ mit m = 0, 1 oder 2; n = 3, 2 oder 1 und m + n = 3 oder 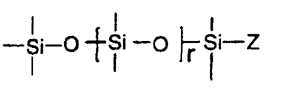 oder 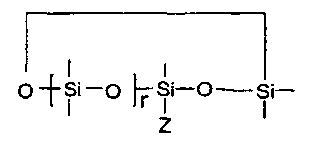 wobei r 0 bis 6 ist.

Für die ausgeprägte UV-A-Filterwirkung scheinen die zwingend 3-fach vorhandenen Hydroxygruppen in ortho-Stellung zum Triazin von wesentlicher Bedeutung zu sein.

Einige Beispiele der erfindungsgemässen Verbindungen sind nachfolgend angeführt (Tabelle I).

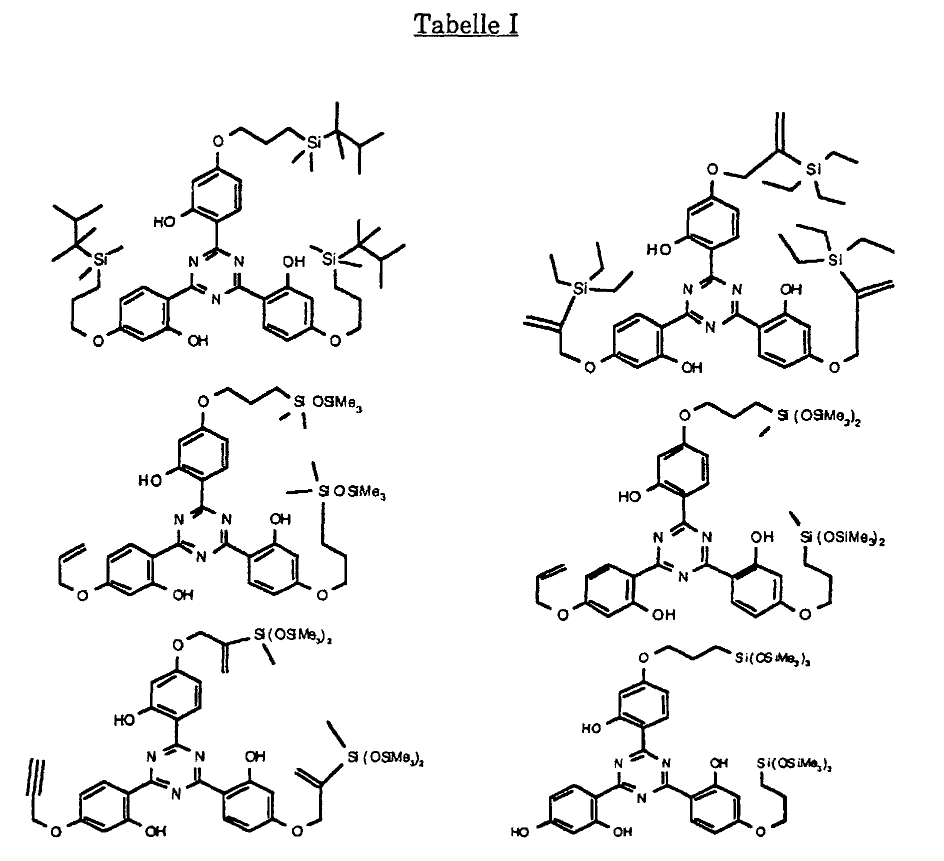

Die erfindungsgemässen Verbindungen sind farblose oder leicht gelbliche, flüssige, kristalline oder halbflüssige Substanzen. Sie zeichnen sich durch hohe Photostabilität, gute Löslichkeit in organischen und insbesondere kosmetischen Lösungsmitteln und durch kurze und billige Zugänge aus.

Die erfindungsgemässen Verbindungen haben ein ausgeprägtes Absorptionsmaximum im UV-A-Bereich. Zusätzlich liegt ein wesentlich kleineres zweites UV-Absorptionsmaximum im UV-B-Bereich, so dass diese Substanzen auch gleichzeitig zum Schutz gegen beide gefährlichen UV-Bereiche eingesetzt werden können.

Um aber einen sog. A+B-Totalblock, der die Haut vor zu früher Alterung und in vielen Fällen vor Lichtdermatosen schützt, zu erhalten, ist es oft vorteilhaft oder sinnvoll, weitere UV-Filter in der gleichen Formulierung einzusetzen, insbesondere andere UV-B-Filter.

Die Herstellung von neuen Lichtschutzmitteln, insbesondere von Hautschutz- bzw. Sonnenschutzpräparaten für die Alltagskosmetik geschieht dadurch, dass man eine Verbindung der Formel I in eine für Lichtschutzmittel übliche kosmetische Grundlage einarbeitet. Bei dieser Einarbeitung kann, falls es zweckmässig ist, auch noch mit weiteren konventionellen UV-A- bzw. UV-B-Filtern kombiniert werden. Um besonders hohe Lichtschutzfaktoren zu erreichen, ist es oft vorteilhaft, mehrere verschiedene UV-Filter gleichzeitig einzusetzen.

Solche UV-A-Filter sind beispielsweise:
1-(4-Methoxyphenyl)-3-(4-tert.-butylphenyl)propan-1,3-dion (=Butylmethoxydibenzoylmethan), Handelsname Parsol® 1789,
4-Isopropyl-dibenzoylmethan,
2,2'-di-Hydroxy-4-methoxybenzophenon (= Dioxybenzon oder Benzophenon-δ),
2-Hydroxy-4-methoxybenzophenon-5-sulfonsäure (= Sulisobenzone, Benzophenone-4),
2-Hydroxy-4-methoxybenzophenon (=Oxybenzone, Benzophenone-3),
3,3,5-Trimethyl-cyclohexyl-N-acetyl-anthranilat (= Homomenthyl-N-acetyl-anthranilat),
2-Methylanthranilat.

Als UV B-Filter, d.h. als Substanzen mit Absorptionsmaxima zwischen etwa 290 und 320 nm, können übliche UV-B-Filter wie beispielsweise die nachstehenden, zu den verschiedensten Stoffklassen gehörenden organischen Verbindungen genannt werden:
1) Derivate der p-Aminobenzoesäure, wie z.B. Aethyl-p-aminobenzoat und andere Ester, wie Propyl-, Butyl-, Isobutyl-p-aminobenzoat, Aethyl-p-dimethylaminobenzoat, Glyceryl-p-aminobenzoat, Amyl-p-dimethylaminobenzoat,
2) Derivate der Zimtsäure, wie z.B. 2-Aethoxyäthyl-p-methoxyzimtsäureester, 2-Aethylhexyl-p-methoxyzimtsäureester, p-Methoxyzimtsäureestergemische, Zimtsäureestergemische,
3) Dibenzalazine,
4) Heterozyklische Stickstoffverbindungen, wie Derivate des 2-Phenylbenzimidazols, z.B. 2-Phenylbenzimidazol-5-sulfonsäure,
5) Derivate der Salicylsäure, z.B. Salicylsäurementhylester, Salicylsäurehomomenthylester, Salicylsäure-phenylester,
6) Derivate des Benzophenons, z.B. 4-Phenylbenzophenon, 4-Phenylbenzophenon-2-carbonsäure-isooctylester, 2-Hydroxy-4-methoxybenzophenon-5-sulfonsäure,
7) Derivate des Cumarins, z.B. 7-Oxycumarin, β-Umbelliferonessigsäure, 6,7-Dioxycumarin,
8) Derivate der Gallussäure, z.B. Digalloyl-trioleat,
9) Arylidencycloalkanone, z.B. Benzylidencampher,
10) Derivate der Anthranilsäure, z.B. Anthranilsäurementhylester,
11) Hydroxyphenylbenztriazol.

Besonders geeignet für eine wirkungsvolle UV-Absorption sind auch Metalloxydnanopigmente wie z.B. Titanoxyd, Zinkoxyd, Ceroxyd, Zirkonoxyd, Eisenoxyd und deren Gemische, die einen mittleren Durchmesser von etwa ≤100 nm besitzen, und die sich mit den erfindungsgemässen filterwirksamen Verbindungen der Formel I sehr gut mischen lassen und zusammen mit den sonstgenannten Filtern ebenfalls sehr wirksam sind.

Als für Lichtschutzmittel übliche kosmetische Grundlage im Sinne der vorliegenden Erfindung kann jede übliche Zubereitung dienen, die den kosmetischen Anforderungen entspricht, z.B. Crèmes, Lotions, Emulsionen, Salben, Gele, Lösungen, Sprays, Sticks, Milch und dgl. Die Lichtschutzwirkung ist natürlich auch von der verwendeten Grundlage abhängig. Die Intensität der Lichtschutzwirkung hängt weiter bei jeder Grundlage von der Wirkstoffkonzentration ab. Geeignete Konzentrationen liegen z.B. zwischen 1-6%, vorzugsweise zwischen 1,5-4% einer Verbindung der Formel I im kosmetischen Präparat. Das Verhältnis von Verbindung I zum gegebenenfalls auch eingebrachten UV-B-Filter ist nicht kritisch.

Auf Grund ihrer guten Lipophilität lassen sich die Verbindungen I gut in öl- und fetthaltige kosmetische Zubereitungen einarbeiten.

Bezüglich der Lipophilität sind die erfindungsgemässen Verbindungen I den eingangs genannten substituierten Triazinen überlegen, indem alle Verbindungen I das im vorliegenden Fall nötige Kriterium, nämlich eine hohe Löslichkeit in kosmetischen Lösungsmitteln wie z.B. Deltyl extra (Isopropylmyristat), Arlanol E (PPG 15-Stearylether), Cétiol LC (Cocoylcapylat/-caprat) oder Crodamol DA (Diisopropyladipat) erfüllen, wie die nachfolgend beispielhaft aufgeführten Verbindungen der Formel I und deren Löslichkeiten in Cetiol LC und/oder in Crodamol DA zeigen (Tabelle II).

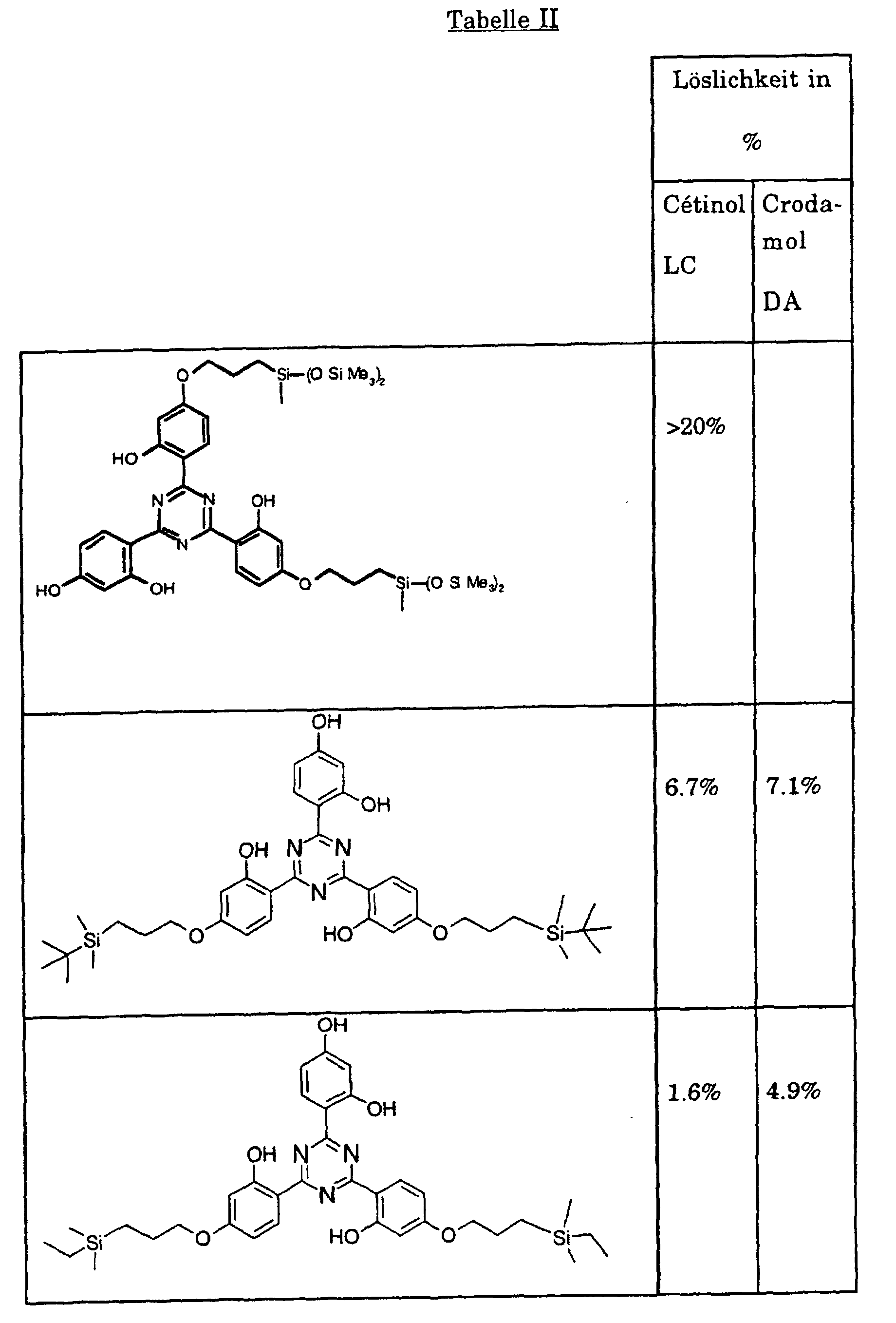

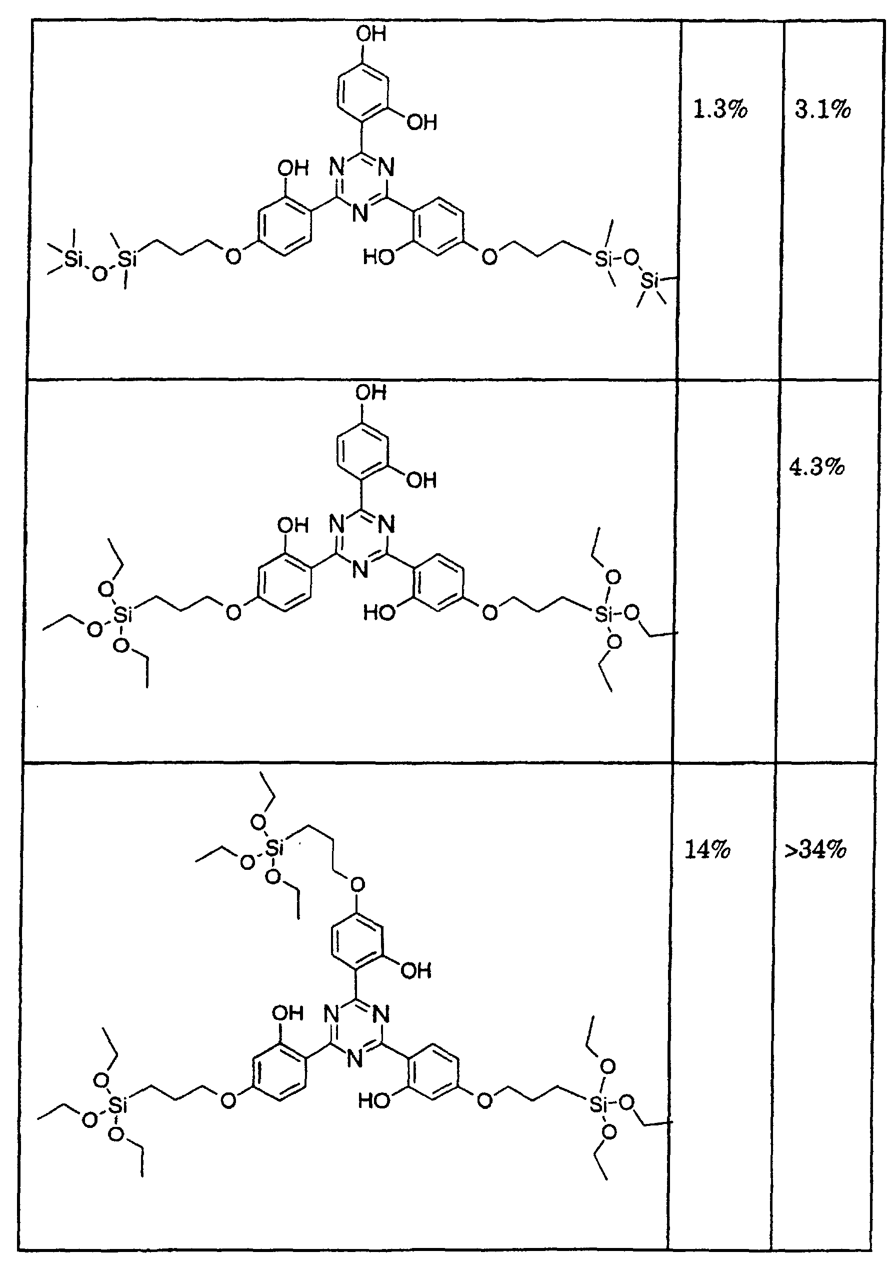

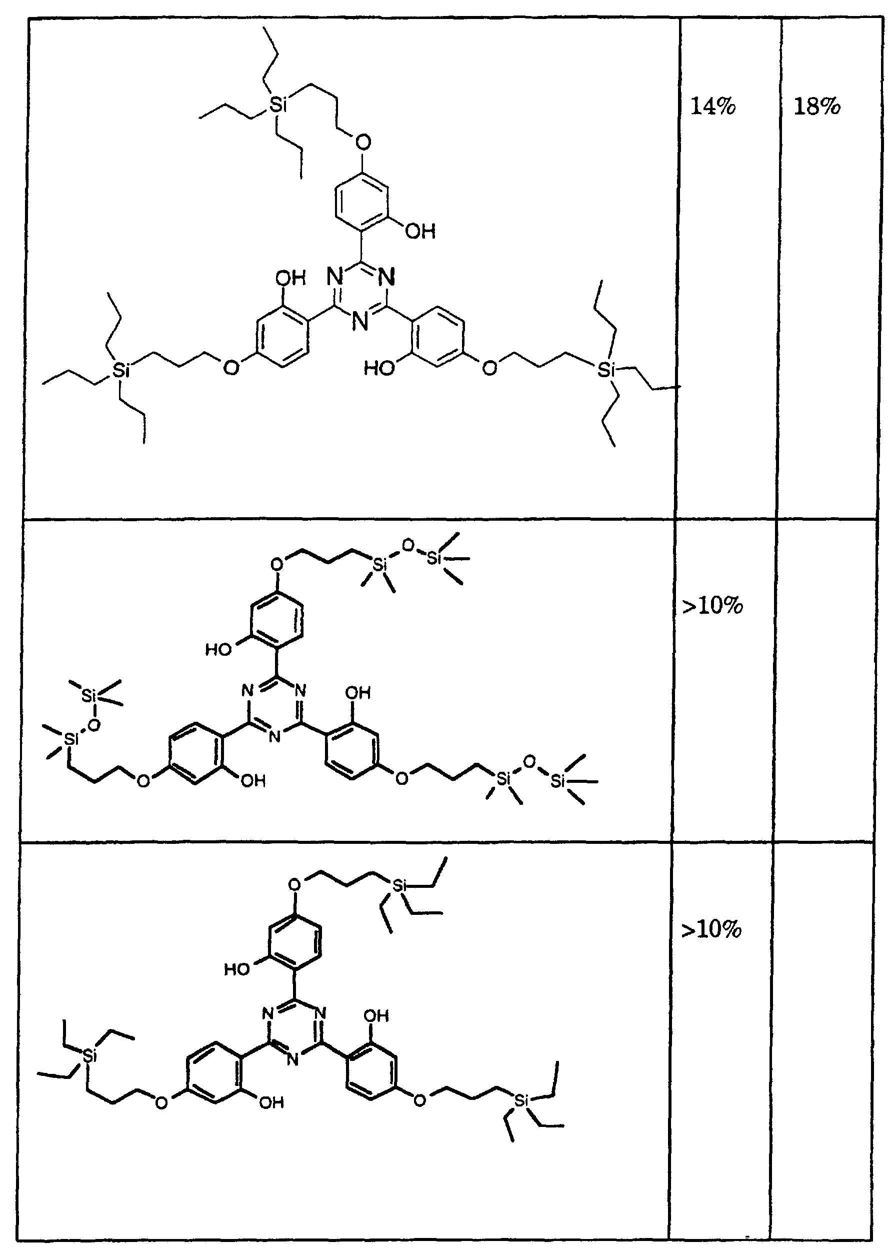

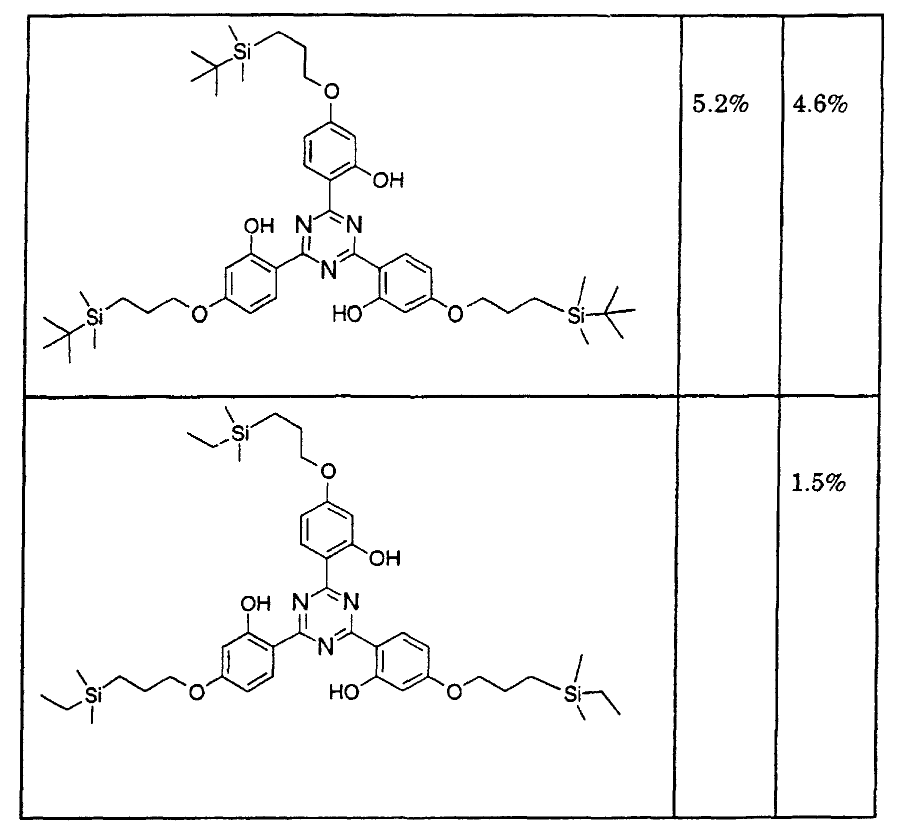

Im Fall der vorbekannten Strukturen werden andererseits in diesen beiden Lösungsmitteln Löslichkeiten von nur 0,5% oder weniger erreicht. Die Vorstufen obiger Verbindungen lösen sich in Cétiol LC zu schlechter als 0.05%.

Die Verbindungen I sind daher besonders für kosmetische Anwendungen geeignet, da sie sowohl in den kosmetischen Lösungsmitteln eine hohe Löslichkeit als auch eine hervorragende UV-A-Filterwirkung aufweisen.

Aus Tabelle II geht ferner hervor, dass bei gleicher Spacergruppe Z die Löslichkeit in den kosmetischen Lösungsmitteln entscheidend von dem Siliziumrest S abhängt, wobei Verbindungen I, an die eine oder mehrere OSiMe₃ oder 3 OEt oder 3 Alkylgruppen grösser als Methyl an Si substituiert sind, die höchsten Löslichkeiten aufweisen und damit derartige Verbindungen I für die Anwendung in Lichtschutzmittel bevorzugt sind.

Die Erfindung richtet sich demgemäss auf die neuen Verbindungen I, deren Herstellung, Lichtschutzmittel, nämlich Lichtschutzpräparate für kosmetische Zwecke mit einem Gehalt an Verbindungen I, vorzugsweise auch in Kombination mit einem UV-B-Filter, und auf die Verwendung der Verbindungen I als Lichtschutzmittel, insbesondere für kosmetische Zwecke.

Der Zugang der erfindungsgemässen Verbindungen I ist in einfacher und billiger Weise möglich und kann wie folgt veranschaulicht werden.

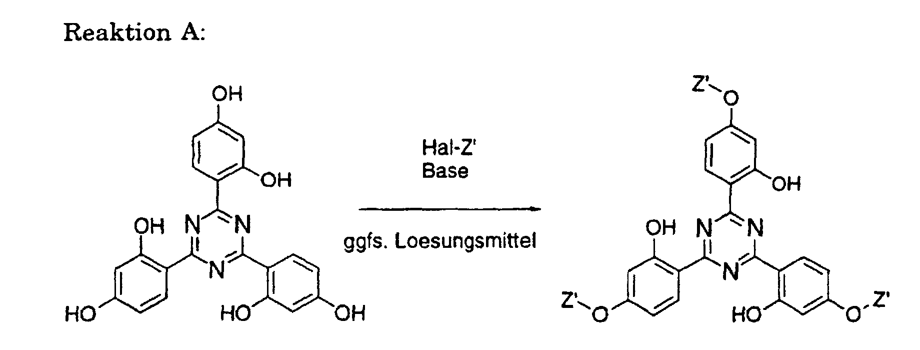

### 1. Stufe

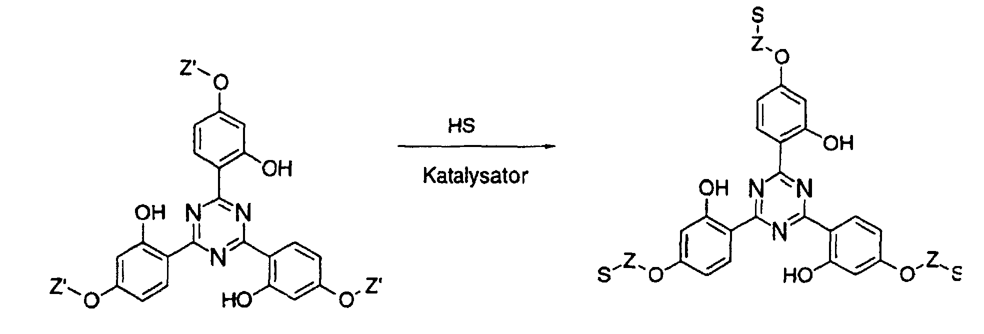

### 2. Stufe

Ausgehend von symmetrischen Tris-(2',4'-dihydroxy phenyl)-1-3-5-triazin werden in einer 1. Stufe zunächst durch Umsetzung mit einer mit einer Abgangsgruppe (leaving group) versehenen Spezies Z', z.B., wie oben in Reaktion A gezeigt, mit einer halogenierten Spezies Hal-Z', in Gegenwart einer Base an den Hydroxyphenylgruppen in 4-Stellung die Spacergruppen Z' substituiert, an die daraufhin in einer 2. Stufe durch Hydrosilylierung mit Si-H-Bindungen die erfindungsgemässe Verbindung I gebildet wird. Dabei unterscheidet sich Z' gegenüber Z darin, dass Z' eine Stufe der Ungesättigtheit mehr enthält als Z. Wenn also z.B. Z eine Doppelbindung enthält, so enthält Z' eine Dreifachbindung oder zwei Doppelbindungen.

Die Reaktion kann jedoch auch nur 1-stufig geführt werden, wenn die mit einer Abgangsgruppe versehene Spezies bereits den Siliziumrest S enthält, d.h. beispielsweise, indem statt Hal-Z' Hal-Z-S verwendet wird, wie dies Reaktion B zeigt.

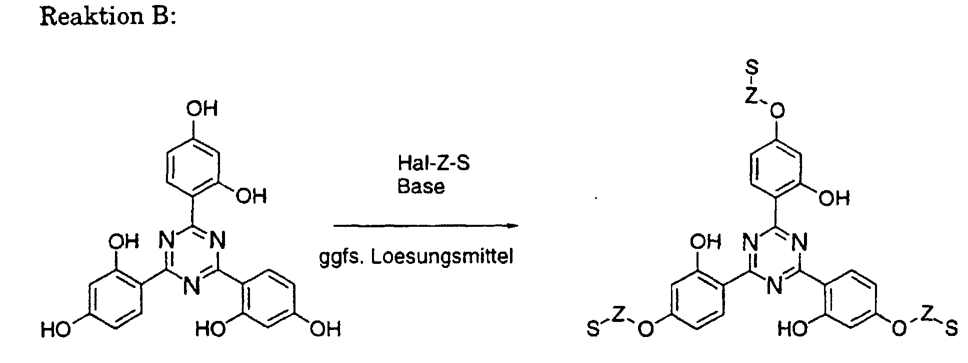

Die 1-stufige Herstellung der Verbindungen der Formel I ist kostengünstiger und daher bevorzugt. Die beiden Reaktionen A und B laufen jedoch oft nicht vollständig ab oder verlieren die Spacergruppe Z während der Reaktion, so dass nach der Umsetzung auch noch Anteile an 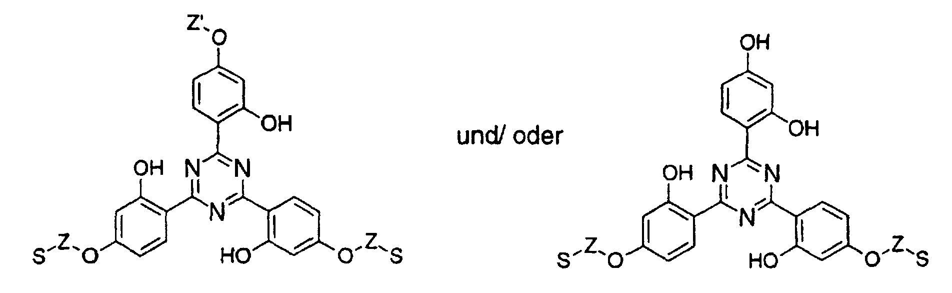 vorhanden sein können.

Als Basen können anorg. Basen wie z.B. KOH, NaOH, NaH oder Alkoholate wie z.B. Na-Methylat, Na-Ethylat, K-tert. Butylat oder Amine wie z.B. Triethylamin, Pyridin, Diazobicyclononan, p-Dimethylaminopyridin oder Amide wie z.B. Li-Diisopropylamid verwendet werden.

Als Lösungsmittel für die erste Stufe der Reaktion A beziehungsweise für den einstufigen Zugang (Reaktion B) eignen sich die gängigen Lösungsmittel, insbesondere polare Lösungsmittel wie Alkohole, z.B. Ethanol, Isopropanol, Methoxyethanol oder Propylenglykol, oder auch THF, Wasser, Pyridin, DMSO oder DMF.

Als Katalysatoren eignen sich homogene Pt-Katalysatoren, z.B. Pt-Divinyl-tetramethyl-disiloxan oder Chloroplatinsäure oder metallisches Platin, z.B. Pt/C. Statt Pt können auch andere Uebergangsmetallkatalysatoren in heterogener Form oder in homogener Form als Komplexe verwendet werden wie z.B. Mo, Ru, Rh, Pd, Cr, Fe, Co, Ni oder Cu. Insbesondere eignen sich Rhodiumsalze oder komplexe wie z.'B. RhodiumIIacetat Dimer, Bis-(1,5-cyclooctadien)-dirhodium(I)-dichlorid, Tris-(triphenylphosphin)-rhodium(I)-dichlorid, Tris-(triphenylphosphin)-rhodium(I)-dichlorid polymer gebunden. Man arbeitet hier entweder ohne Lösungsmittel, oder in Lösungsmitteln, z.B. in Toluol, Xylol, Pyridin, THF, Dioxan, Dichlorethan oder Methylenchlorid.

Weitere Vorteile, Merkmale und Einzelheiten ergeben sich aus der nachfolgenden Beschreibung von Beispielen.

### Beispiel 1

### Einführung von Triethyl-silan in 5,5',5"-tris-Allyloxy-2,2',2"-[1.3.5]-triazin-2,4,6-triyltris-phenol zu 5,5',5"-tris-(3-Triethylsilyl)-propyloxy-2,2',2"-[1.3.5]-triazin-2,4,6-triyl-tris-phenol

1.74 g (3 Aequiv.) Triethyl-silan (Fluka) werden zu einer Suspension von 2,63 g 5,5',5"-tris-Allyloxy-2,2',2"-[1.3.5]-triazin-2,4,6-triyl-tris-phenol, hergestellt gemäss Patent CH 484 695, in 35 ml Toluol unter Ar-Schutzgas zugetropft, mit einem Tropfen Platin-divinyl-tetramethyl-disiloxan Lösung (ABCR GmbH) versetzt, und während 100 Stunden auf 80°C. erhitzt. Die erkaltete Lösung wird darauf mit feuchtem Methanol gewaschen und eingeengt und ergibt 3.7 g eines honigartigen Rohproduktes, welches zur Identifikation in Hexan über Kieselgel chromatographiert wird. Ausbeute 40% der Theorie einer gelblichen klebrigen Masse, UV 362 nm (76'090/E= 870).

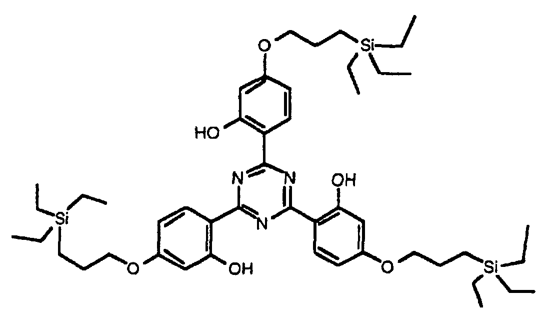

### Beispiel 2

### Einführung von 1,1,1,3,5,5,5-Heptamethyl-trisiloxan in 5,5',5"-tris-Allyoxy-2,2'2"-[1.3.5]-triazin-2,4,6-triyl-tris-phenol

3.3 g (3 Aequivalente) 1,1,1,3,5,5,5-Heptamethyl-trisiloxan (Fluka) werden zu einer Suspension von 2,6 g 5,5',5"-tris-Allyloxy-2,2'2"-[1.3.5]-triazin-2,4,6-triyl-tris-phenol hergestellt gemäss Patent CH 484 695, in 40 ml Toluol unter Ar-Schutzgas zugetropft, mit einem Tropfen Platin-divinyl-tetramethyl-disiloxan Lösung (ABCR GmbH) versetzt, und während 20 Stunden auf 90° C erhitzt. Die erkaltete Lösung wird mit feuchtem Methanol gewaschen und eingeengt und ergibt 4,2 g des dickflüssigen Rohproduktes, welches zur Identifikation in Hexan / Essig-ethylester (im Verhältnis 19:1) über Kieselgel chromatographiert wird. Ausbeute 54% der Theorie eines gelblichen Oels, UV 362 nm (E=630).

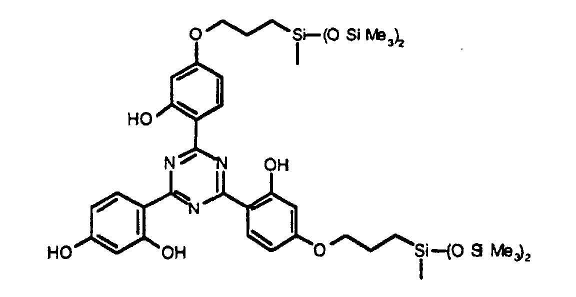

### Beispiel 3

### Einführung von 1,1,1,3,3-Penthamethyl-disiloxan in 5,5',5"tris-Allyloxy-2,2',2"-[1.3.5]-triazin-2,4,6-triyl-tris-phenol

2.6 g 5,5'5"-tris-Allyloxy-2,2'2"-[1.3.5]-triazin-2,4,6-triyl-tris-phenol wird in gleicher Weise wie in Beispiel 1 mit 3 Aequivalenten 1,1,1,3,3-Pentamethyl-disiloxan (Fluka) anstelle von Heptamethyl-trisiloxan während 3 Stunden bei 70° C umgesetzt und aufgearbeitet. Das Rohprodukt wird in Methylenchlorid aufgenommen, über einen Kieselgel-Pfropfen filtriert und eingeengt. Es werden 2.8 g eines dickflüssigen, klaren Oels erhalten. UV 362 nm (E = 808).
Behandelt man das gleiche Reaktionsgemisch ohne Toluol als Lösungsmittel während 7.5 Stunden bei 80° C und arbeitet in gleicher Weise auf, so erhält man dasselbe Produkt in 85% Ausbeute.
Das Produkt erhält durchschnittlich 2.55 Aequivalente Spacergruppen.

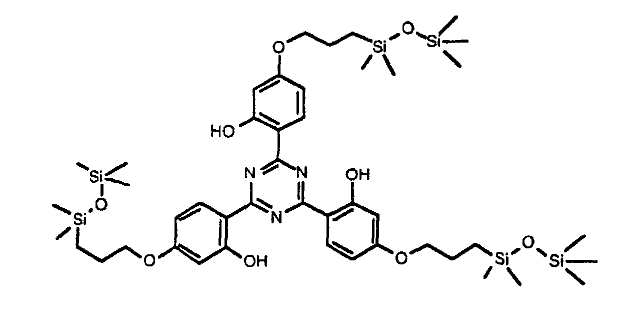

### Beispiel 4

### Einführung von tris-Trimethyl-silyloxy-silan in 5,5',5"-tris-Allyloxy-2,2'2"-[1.3.5]-triazin-2,4,6-triyl-tris-phenol

2.6 g 5,5'5"-tris-Allyloxy-2,2',2"[1.3.5]-triazin-2,4,6-triyl-tris-phenol wird in gleicher Weise wie in Beispiel 1 mit 3 tris-Trimethyl-silyloxy-silan (Wacker) anstelle von Heptamethyl-trisiloxan während 6 Tagen unter Rückflusstemperatur umgesetzt und aufgearbeitet. Die erhaltenen 4.8 g Rohprodukt werden in Hexan / Essig-ethylester im Verhältnis 19:1 über Kieselgel chromatographiert. Man erhält 1.9 g einer dickflüssigen, klebrigen Masse, UV 360 nm (E = 466), und enthält 1.9 Aequivalente Spacergruppen.

Das gleiche Produkt wird erhalten, wenn man 0.53 g 5,5',5"-tris-Allyloxy-2,2',2"-[1.3.5]-triazin-2,4,6-triyl-tris-phenol und 0.98 g tris-Trimethyl-silyloxy-silan in 9 ml Xylol mit 0.02 g Pt (10%) auf Aktivkohle versetzt, während 6 Tagen rückflussiert und in gleicher Weise aufarbeitet.

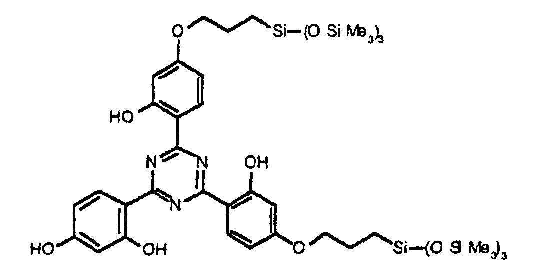

### Beispiel 5

### Einführung von Triethoxysilan in 5,5'5"-tris-Allyloxy-2,2',2"-[1.3.5]-triazin-2,4,6-triyl-tris-phenol

2.6 g 5,5',5"-tris-Allyloxy-2,2',2"-[1.3.5]-triazin-2,4,6-triyl-tris-phenol wird in gleicher Weise wie in Beispiel 2 mit 3 Aequivalenten Triethoxysilan anstelle von Heptamethyl-trisiloxan während 3 Stunden bei 80° C umgesetzt und aufgearbeitet. Man erhält 92% Rohausbeute.

Dieses Produkt wird in Hexan / Ethylacetat im (Verhältnis 9:1) chromatographiert. Man erhält 50% der Theorie an 3-fach substituiertem Triazin (A), UV 362 nm (E = 779, Schmelzpunkt 71-73° C, sowie 17% der Theorie an 2-fach substituiertem Triazin (B), (UV 360 nm (E 824) Schmelzpunkt 153-155° C.).

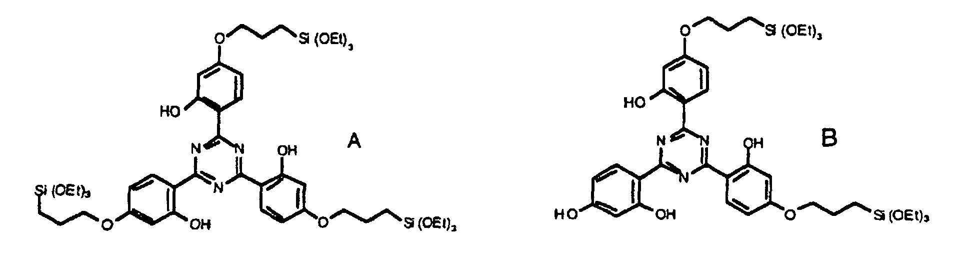

### Beispiel 6

### Einführung von Triethylsilan in 5,5',5"-tris-Propargyloxy-2,2',2"-[1.3.5]triazin-2,4,6-triyl-tris-phenol

a) Herstellung von 5,5',5"-tris-Propargyloxy-2,2',2"-[1.3.5]-triazin-2,4,6-triyl-tris-phenol:
   50 g 2,4,6-tris-(2,4-Dihydroxyphenyl)-1,3,5-triazin (hergestellt gemäss Patent, CH 484'695 ) und 41,4 g Kalium tert. butylat werden in 500 ml Methoxyethanol suspendiert und bei 55°C mit 44.3 g Propargylbromid versetzt. Das Reaktionsgemisch wird 2 Tage bei dieser Temperatur gerührt, abgekühlt, abgenutscht und den Filterrückstand mit Ether nachgewaschen. Man erhält 48.6 g (76%) des gewünschten Produktes, UV 348 nm ( E = 1123), Schmelzpunkt >240°C.
b) 0.7 g Triethylsilan (Fluka) werden zu einer Suspension von 0.51 g 5, 5',5"-tris-Propargyloxy-2, 2',2"-[1.3.5]-triazin-2,4,6-triyl-tris-phenol in 18 ml Dimethylformamid unter Ar-Schutzgas zugegeben, mit einem Tropfen Platin-divinyl-tetramethyl-disiloxan Lösung (ABCR GmbH,) versetzt, und während 7 Stunden auf 85°C. erhitzt. Die erkaltete Lösung wird zwischen Essigester und Wasser verteilt. Die organische Phase wird über einen Kieselgelpfropfen filtriert und eingeengt und ergibt eine harzige Masse des Produktes. UV 352 nm (E= 627).

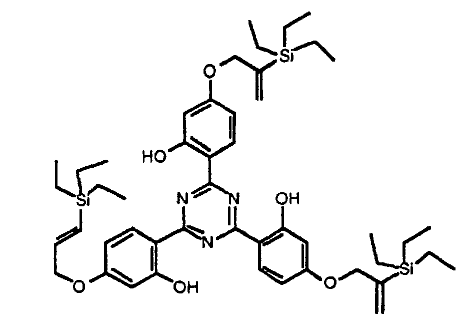

### Beispiel 7

### Einführung von 1,1,1,3,3-Pentamethyl-disiloxan in 5,5',5"-tris-(But-2-enyl-1-oxy)-2,2',2"-[1.3.5]-triazin-2,4,6-triyl-tris-phenol

a) Herstellung von 5,5',5"-tris-(But-2-enyl-1-oxy)-2,2',2"-[1.3.5]-triazin-2,4,6-triyl-tris-phenol:
   6 g 2,4,6-tris-(2,4-Dihydroxyphenyl)-1,3,5-triazin (hergestellt nach Patent CH 484'695) und 5 g Kalium tert. butylat werden in 150 ml Methoxyethanol suspendiert und bei 55°C mit 5.4 g trans-Crotylbromid versetzt, etwa 12 Stunden auf 65°C erhitzt, abgekühlt, eingeengt und in Hexan/Essigester (im Verhältnis 19:1) über Kieselgel chromatographiert. Das Frontprodukt wird isoliert. Man erhält 2.1 g (25%) eines gelben Pulvers, UV 362 nm ( E = 1080), Schmelzpunkt 133 - 137°C.
b) 0.45 g (3 Aequivalente) 1,1,1,3,5,5,5-Heptamethyl-trisiloxan (Fluka) werden zu einer Suspension von 0.57 g 5, 5',5"-tris-(But-2-enyl-1-oxy)-2, 2',2"-[1.3.5]-triazin-2,4,6-triyl-tris-phenol in 15 ml Toluol unter Ar-Schutzgas zugegeben, mit einem Tropfen Platin-divinyl-tetramethyl-disiloxan Lösung (ABCR GmbH) versetzt und etwa 12 Stunden auf 80°C erhitzt. Die erkaltete Lösung wird mit feuchtem Methanol gewaschen, eingeengt und im Hochvakuum getrocknet. Man erhält 0.85 g eines dickflüssigen Rohproduktes. Es handelt sich um ein Gemisch mit an verschiedenen Stellen substituierten Silylgruppen.

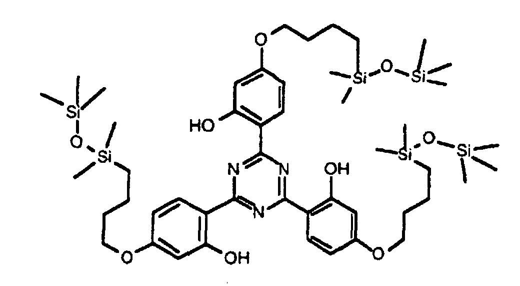

### Beispiel 8

### Einführung von tert.-Butyl-dimethylsilan in 5, 5',5"-tris-Allyloxy-2, 2',2"-[1.3.5]-triazin-2,4,6-triyl-tris-phenol

3.9 g 5,5',5"-tris-Allyloxy-2,2',2"-[1.3.5]-triazin-2,4,6-triyl-tris-phenol wird in gleicher Weise wie in Beispiel 2 mit 3 Aequivalenten tert.-Butyldimethylsilan anstelle von Heptamethyl-trisiloxan und in Dichlorethan statt Toluol während 3 Tagen bei 75°C umgesetzt und aufgearbeitet. Man erhält 80% Rohausbeute.

Dieses Rohprodukt wird in Hexan / Ethylacetat im (Verhältnis 9:1) chromatographiert. Man enthält 21% der Theorie an 3-fach substituierten Triazin (C), (UV 362 nm (E = 801)/ Schmelzpunkt 154-157°C.), sowie 46% der Theorie an 2-fach substituierten Triazin (D), UV 362 nm (E = 885), Schmelzpunkt 143-148°C.

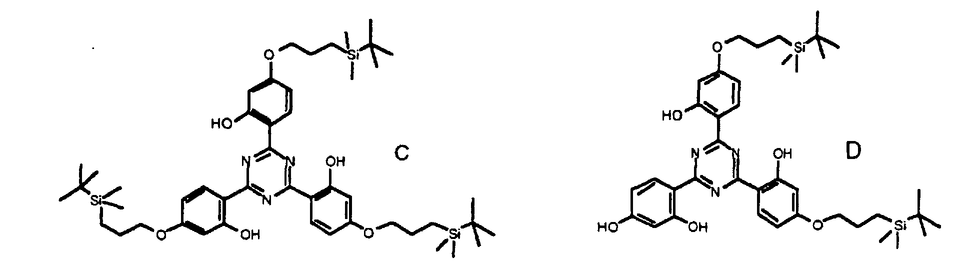

### Beispiel 9-1

### Einführung von 1,1,1,3,3-Pentamethyl-disiloxan in 5,5',5"-tris-(Pent-4-enyl-1-oxy)-2,2',2"-(1.3.5)triazin-2,4,6-triyl-tris-phenol

a) Herstellung von 5,5',5"-tris-(Pent-4-enyl-1-oxy)-2,2',2"-(1.3.5)triazin-2,4,6-triyl-tris-phenol: 6 g 2,4,6-tris-(2,4-Dihydroxyphenyl)-1,3,5-triazin (hergestellt nach Patent CH 484'695) werden in einer Lösung von 2.5 g Kalium tert. butylat in 60 ml Ethanol suspendiert und mit 5.3 g 5-Brom-1-penten versetzt. Die klare, orange-rote Lösung wird während 72 Stunden bei 70°C gerührt, auf 0°C abgekühlt, über eine Filternutsche abfiltriert und mit 150 ml Wasser und 100 ml kaltem Ethanol nachgewaschen. Der Filterrückstand wird unter Hochvakuum getrocknet. Man erhält 7.5 g (82%) eines gelben Pulver. UV 361 nm (E = 1080), Schmelzpunkt 140-143°C.
b) 2.2 g (3 Aequivalente) 1,1,1,3,3-Pentamethyl-disiloxan (Fluka) werden zu einer Suspension von 3 g 5,5',5"-tris-(Pent-4-enyl-1-oxy)-2,2',2"-(1.3.5)-triazin-2,4,6-triyl-tris-phenol in 40 ml 1,2-Dichlorethan unter Ar-Schutzgas zugegeben, mit einem Tropfen Platin-divinyl-tetramethyl-disiloxan Lösung (ABCR GmbH) versetzt und über Nacht bei 70°C gerührt. Die erkaltete Lösung wird auf 100 ml Wasser gegossen, 2 mal mit 50 ml Dichlormethan nachgewaschen, die vereinigten organischen Phasen eingeengt und unter Hochvakuum getrocknet. Man erhält 4.9 g eines dickflüssigen, gelbbraunen Rohproduktes. Durch Chromatographie in Hexan/Ethylacetat (Verhältnis 95:5) erhält man 2.65 g eines wachsartigen Materials mit einer Löslichkeit in Cétinol LC von >35%. UV 362 nm (E = 736), Schmelzpunkt 61-63°C. 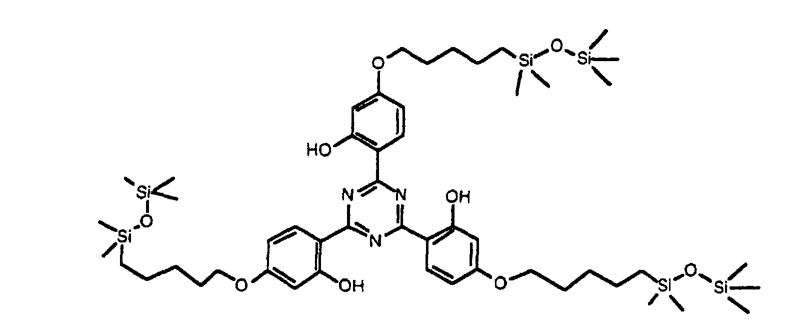 (5,5',5''-Tis-[5-(1,1,1,3,3-pentamethyl-disiloxan-oxy)-pentyl-1-oxy]-2,2',2''-(1.3.5)-triazin-2,4,6-triyl-tris-phenol)

### Beispiel 9-2

### Einführung von 1-(5-Bromo-pentyl)-1,1,3,3,3-pentamethyl-disiloxan in 2,4,6-tris-(2,4-Dihydrophenyl)1,3,5-triazin.

### einstufige Reaktionsführung

a) Herstellung von 1-(5-Bromo-pentyl)-1,1,3,3,3-pentamethyl-disiloxan.
   Zu einer Lösung von 3,9 ml Pentamethyl-disiloxan und 0.01 ml Platindivinyl-tetramethylsiloxan-komplex in 10 ml 1,2 Dichlorethan werden unter Inertgas 2,4 ml 5-Brom-1-penten zugetropft und über Nach bei 60 °C gerührt. Darauf wird das Reaktionsgemisch zwischen Wasser und CH₂Cl₂ verteilt, die organische Phase über Na₂SO₄ getrocknet, eingeengt und bei 110°C/0.06 mbar destilliert. Man erhält 3.8 g einer farblosen Flüssigkeit, deren Identität mittels GC,NMR und MS bestätigt wird.
b) Verätherung:
   0.81 g 2,4,6-tris-(2,4-Dihydroxyphenyl)-1,3,5-triazin werden in einer Lösung von 0.34 g KOH in 10 ml Ethanol bei 60 °C gelöst und unter Argon mit 1.78 g 1-(5-Bromo-pentyl)-1,1,3,3,3-pentamethyl-disiloxan versetzt. Die Reaktion wird über Nacht bei 70 °C gerührt, mit 18 ml Tetrahydrofuran versetzt, und nochmals 48 Stunden bei 70 °C weiter gerührt. Die Reaktionslösung wird anschliessend zwischen Wasser und CH₂Cl₂ verteilt, die eingeengte organische Phase in Hexan/ EtOAc (97:3-93:7) über Silikagel chromatographiert und die Frontfraktion eingeengt. Man erhält 0.75 g des gewünschten Produktes als gelbliches Pulver, welches mit dem Produkt aus Beispiel 9-1 identisch ist.

### Beispiel 10

### Einführung von 1,1,1,3,3-Pentamethyl-disiloxan in 5,5',5"-tris-(But-3-enyl-1-oxy)-2,2',2"-(1.3.5)triazin-2,4,6-triyl-tris-phenol

a) Herstellung von 5,5',5"-tris-(But-3-enyl-1-oxy)-2,2',2"-(1.3.5)triazin-2,4,6-triyl-tris-phenol: 2,4,6-tris-(2,4-Dihydroxyphenyl)-1,3,5-triazin wird in gleicher Weise, wie es in Beispiel 9a beschrieben ist, mit 4-Brom-1-buten statt mit 5-Brom-1-penten umgesetzt. Man erhält ein hellgelbes Pulver in 60% Ausbeute. UV 361 nm (E = 1258), Schmelzpunkt 166-169°C.
b) Das oben erhaltene Material wird in gleicher Weise wie in Beispiel 9b beschrieben mit 1,1,1,3,3-Pentamethyl-disiloxan (Fluka) umgesetzt. Man erhält ein gelbgrünes, halbfestes Rohprodukt. Durch Chromatographie in Hexan/Ethylacetat (Verhältnis 95:5) wird ein hellgelber Feststoff erhalten mit einer Löslichkeit in Cétinol LC von 8%. UV 362 nm (E = 933), Schmelzpunkt 72-75°C. 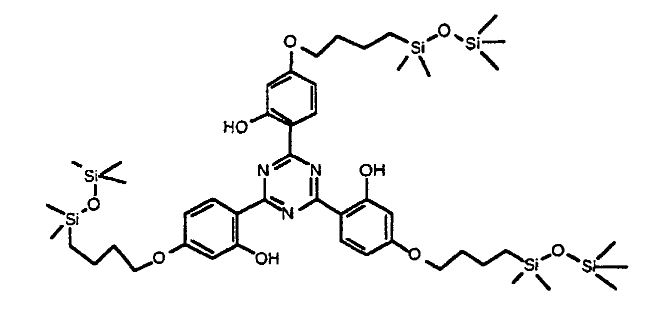 (5,5',5"-Tris-[4-(1,1,1,3,3-pentamethyl-disiloxan-oxy)-butyl-1-oxy]-2,2',2"-(1.3.5)-triazin-2,4,6-triyl-tris-phenol)

### Beispiel 11

### Einführung von 1,1,1,3,3-Pentamethyl-disiloxan in 5,5',5"-tris-(Hex-5-enyl-1-oxy)-2,2',2"-(1.3.5)triazin-2,4,6-triyl-tris-phenol

a) Herstellung von 5,5',5"-tris-(Hex-5-enyl-1-oxy)-2,2',2"-(1.3.5)triazin-2,4,6-tris-phenol: 2,4,6-tris-(2,4-Dihydroxyphenyl)-1,3,5-triazin wird in gleicher Weise wie in Beispiel 9-1a beschrieben mit 6-Brom-1-hexen statt mit 5-Brom-1-penten umgesetzt. Man erhält ein hellgelbes Pulver in 79% Ausbeute. UV 361 nm (E = 1032), Schmelzpunkt 155-158°C.
b) Das oben erhaltene Material wird in gleicher Weise, wie in Beispiel 9-1b beschrieben mit 1,1,1,3,3-Pentamethyl-disiloxan (Fluka) umgesetzt. Man erhält ein braunes, dickflüssiges Rohprodukt. Durch Chromatographie in Hexan/Ethylacetat (Verhältnis 95:5) wird ein hellgelber Feststoff erhalten. UV 360 nm (E = 725). 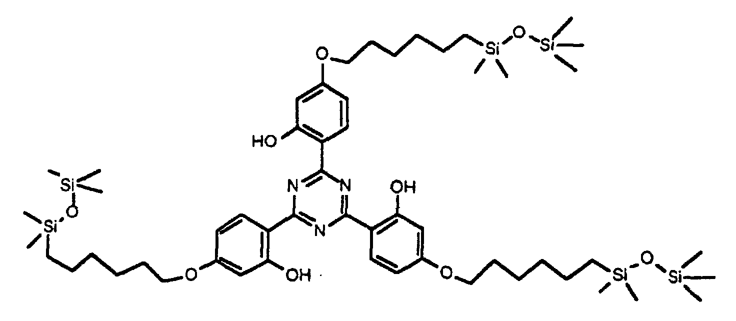 (5,5',5"-Tris-[6-(1,1,1,3,3-pentamethyl-disiloxan-oxy)-hexyl-1-oxy]-2,2',2"-(1.3.5)-triazin-2,4,6-triyl-tris-phenol)

### Beispiel 12

### Herstellung einer O/W Sonnenschutz Lotion UV-A und UV-B: Breitspektrum Sonnenschutz-Lotion mit 3% UV-Filter aus Beispiel 1

### Rezept:

| | **%** | **Bestandteil** | **chemischer Name** |
|---|---|---|---|
| **A** | 6 | Parsol MCX | Octyl Methoxycinnamat |
| | 3 | Produkt aus Beisp. 1 | |
| | 10 | Cétiol LC | Coco-Caprylat/Caprat |
| | 4 | Dermol 185 | Isostearyl neopentanoat |
| | 0.25 | Diethylenglycol | PEG-2-stearat |
| | | monostearat | |
| | 1 | Cetylalkohol | Cetylalkohol |
| | 0.25 | MPOB/PPOB | Methyl-Propylparaben |
| | 0.1 | EDTA BD | EDTA-Na-Salz |
| | 1 | Amphisol DEA (Giv.) | Diethanolamin Cetylphosphat |
| **B** | 20 | Permulen TR-1 (+%) | Acrylat C10-C30 Alkylacrylat |
| | 48.6 | Wasser deionisiert | Aqua |
| | 5 | Propylenglykol | 1,2-Propandiol |
| | 0.8 | KOH (10%) | Kaliumhydroxyd |

### Herstellung: Erhitzen von A auf etwa 85°C im Reaktor, Erhitzen von B auf etwa 85°C auf einer Heizplatte gemäss unteren Angaben

**Reaktor:** 250 ml **Ansatzgrösse:** 100 g

| **Zeit min** | **Rühren Umdreh ungen/ Minute** | **Bad °C** | **Kühlen** | **Vacuum** | **Produkt °C** | **Bemerkungen und Operationen** |
|---|---|---|---|---|---|---|
| | | | **ja/nein** | **ja/nein** | | |
| 0 | 200 | 90 | n | n | RT | Teil A |
| 25 | 500 | 90 | n | n | 69.5 | Zugabe von Teil B, 7.8ml/min |
| 35 | 500 | 90 | n | n | 84.3 | Ende der Zugabe |
| 45 | 200 | 20 | j | n | 85.8 | Zugabe von KOH, Kühlen |
| 75 | 100 | 20 | j | j | 21.5 | Entgasen |
| 85 | 40 | 20 | j | j | 21 | Entgasen |
| 95 | ∼ | ∼ | ∼ | ∼ | 20.9 | Ende der Emulgierung |

## Patentansprüche

1. Verbindung der Formel I, 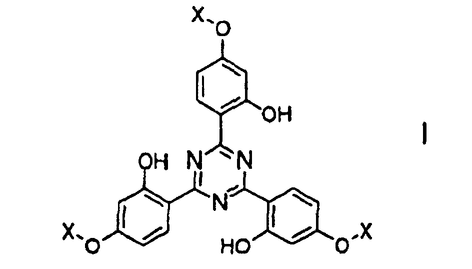 worin alle X über Spacergruppen Z an O gebundene Siliziumreste S sind oder zwei X über Spacergruppen Z an O gebundene Siliziumreste S sind und das dritte X H oder ein an O gebundener Siliziumrest S ist,
wobei die Spacergruppe Z eine gesättigte, einfach oder mehrfach ungesättigte Alkylgruppe mit 3-12 C-Atomen ist und
der Siziliumrest S 1-8 Si-Atome enthält und aus 3-fach substituierten Alkyl-silylgruppen, Alkoxy-silylgruppen mit je 3-15 C-Atomen oder einem Oligosiloxan besteht.

2. Verbindung der Formel I gemäss Anspruch 1, worin alle X über Spacergruppen Z an O gebundene Siliziumreste S sind.

3. Verbindung der Formel I gemäss Anspruch 1 oder 2, wobei die Alkylgruppen der Spacergruppen Z in Stellung 2 oder 3 mit den Siliziumresten S verbunden sind.

4. Verbindung der Formel I gemäss Anspruch 1 oder 2, wobei die Alkylgruppen der Spacergruppen Z in Stellung 4, 5 oder 6 mit den Siliziumresten S verbunden sind.

5. Verbindung der Formel I gemäss einem der Ansprüche 1 bis 4, **dadurch gekennzeichnet, dass** die Spacergruppen Z 3-Propyl-, 2-Propyl-, 3-Propenyl-, 2-Propen-3-yl-, 2-Propen-2-yl-, 2-Methyl-2-propyl, 2-Methyl-3-propenyl, 4-Butyl, 3-Butyl-, 2-Butyl-, 3-Butenyl-, 3-Methyl-2-butyl-, 2,4-Pentadien-5-yl-, 4-Pentyl-, 5-Pentyl-, 5-Hexyl-, 6-Hexyl- oder 2-Dodecen-12-yl-; vorzugsweise 3-Propyl-, 4-Butyl oder 5-Pentylsind.

6. Verbindung der Formel I gemäss einem der Ansprüche 1 bis 5, **dadurch gekennzeichnet, dass** die Siliziumreste S -Si-Me₃, -Si-Et₃, -Si-Isopropenyl₃-, -Si-tert.Butyl₃, -Si-sec.Butyl₃, -Si-Me₂-tert.Butyl, -Si-Me₂-Thexyl, -Si(OMe)₃, -Si(OEt)₃, -Si(OPh)₃ oder Oligosiloxan; vorzugsweise -Si(OEt)₃ oder SiEt₃ sind.

7. Verbindung der Formel I gemäss Anspruch 6, **dadurch gekennzeichnet, dass** das Oligosiloxan -SiMeₘ(OSiMe₃)ₙ mit m = 0, 1 oder 2; n = 3, 2 oder 1 und m + n = 3; vorzugsweise -SiMe₂OSiMe₃ ist oder ein Oligosiloxan der Formeln 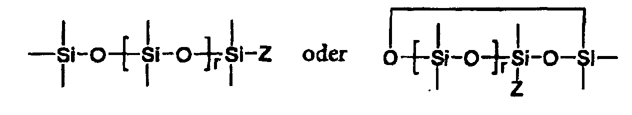 bedeutet, wobei r 0 bis 6 ist

8. Verbindung der Formel I gemäss Anspruch 1:
5,5',5"-Tris-(3-triethylsilyl)-propyloxy-2,2',2"-[1.3.5]-triazin-2,4,6-triyl-tris-phenol;
5,5'-Di-[3-(1,1,1,3,5,5,5-heptamethyl-trisilyl)-propyloxy]-5"-oxy-2,2',2"-[1.3.5]-triazin-2,4,6-triyl-tris-phenol;
5,5',5"-Tris-[3-(1,1,1,3,3-pentamethyl-trisilyl)-propyloxy]-2,2',2"-[1.3.5]-triazin-2,4,6-triyl-tris-phenol;
5,5'-Di-[tris-(3-trimethyl-siloxy-silyl)-propyloxy]-5"-oxy-2,2',2"-[1.3.5]-triazin-2,4,6-triyl-tris-phenol;
5,5',5"-Tris-[3-triethoxysilyl-propyloxy]-2,2',2"-[1.3.5]-triazin-2,4,6-triyl-tris-phenol;
5,5'-Di-[3-triethoxysilyl-propyloxy]-5"-oxy-2,2',2"-[1.3.5]-triazin-2,4,6-triyl-trisphenol;
5,5',5"-Tris-[2-(triethylsilyl)-2-methylen-ethoxy]-2,2',2"-[1.3.5]-triazin-2,4,6-triyl-trisphenol;
5,5',5"-Tris-[4-(1,1,1,3,3-pentamethyl-trisilyl)-butyloxy]-2,2',2"-[1.3.5]-triazin-2,4,6-triyl-tris-phenol;
5,5',5"-Tris-[3-(tert. butyl-dimethyl-silyl)-propyloxy]-2,2',2"-[1.3.5]-triazin-2,4,6-triyltris-phenol;
5,5'-Di-[3-(tert. butyl-dimethyl-silyl)-propyloxy]-5"-oxy-2,2',2"-[1.3.5]-triazin-2,4,6-triyl-tris-phenol;
5,5',5"-Tris-[5-(1,1,1,3,3-pentamethyl-disiloxan-oxy)-pentyl-1-oxy]-2,2',2"-(1.3.5)-triazin-2,4,6-triyl-tris-phenol;
5,5',5"-Tris-[4-(1,1,1,3,3-pentamethyl-disiloxan-oxy)-butyl-1-oxy)-2,2',2"-(1.3.5)-triazin-2,4,6-triyl-tris-phenol;
5,5',5"-Tris-[6-(1,1,1,3,3-pentamethyl-disiloxan-oxy)-hexyl-1-oxy]-2,2',2"-(1.3.5)-triazin-2,4,6-triyl-tris-phenol.

9. Lichtschutzmittel, **gekennzeichnet durch** einen Gehalt an mindestens einer Verbindung der Formel I gemäss mindestens einem der Ansprüche 1 bis 8 und gegebenenfalls noch an mindestens einem weiteren UV-Filter.

## Claims

1. A compound of formula I 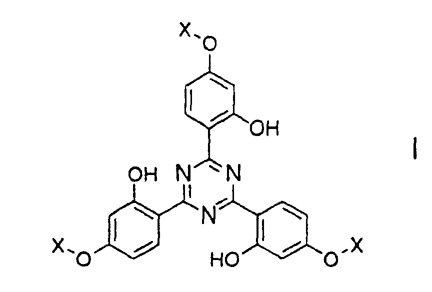 wherein all X's are silicon residues S bonded to O via spacer groups Z or two X's are silicon residues S bonded to O via spacer groups Z and the third X is H or a silicon residue S bonded to O,
in which the spacer group Z is a saturated or mono- or multiply-unsaturated alkyl group with 3-12 C atoms and
the silicon residue S contains 1-8 Si atoms and consists of trisubstituted alkylsilyl or alkoxy-silyl groups with in each case 3-15 C atoms or an oligosiloxane.

2. A compound of formula I according to claim 1, wherein all X's are silicon residues S bonded to O via spacer groups Z.

3. A compound of formula I according to claim 1 or claim 2, wherein the alkyl groups of the spacer Z are bonded in position 2 or 3 with the silicon residues S.

4. A compound of formula I according to claim 1 or claim 2, wherein the alkyl groups of the spacer groups Z are bonded in position 4, 5 or 6 with the silicon residues S.

5. A compound of formula I according to any one of claims 1 to 4, wherein the spacer groups Z are 3-propyl, 2-propyl, 3-propenyl, 2-propen-3-yl, 2-propen-2-yl, 2-methyl-2-propyl, 2-methyl-3-propenyl, 4-butyl, 3-butyl, 2-butyl, 3-butenyl, 3-methyl-2-butyl, 2,4-pentadien-5-yl, 4-pentyl, 5-pentyl, 5-hexyl, 6-hexyl or 2-dodecen-12-yl, preferably 3-propyl, 4-butyl or 5-pentyl.

6. A compound of formula I according to any one of claims 1 to 5, wherein the silicon residues S are -Si-Me₃, -Si-Et₃, -Si-isopropenyl₃, -Si-tert.butyl₃, -Si-sec.butyl₃, -Si-Me₂-tert.butyl, -Si-Me₂-thexyl, -Si(OMe)₃, -Si(OEt)₃, -Si(OPh)₃ or oligosiloxane; preferably -Si(OEt)₃ or SiEt₃.

7. A compound of formula I according to claim 6, wherein the oligosiloxane is -SiMeₘ(OSiMe₃)ₙ with m = 0, 1 or 2, n = 3, 2 or 1 and m + n = 3, preferably -SiMe₂OSiMe₃, or an oligosiloxane of the formula 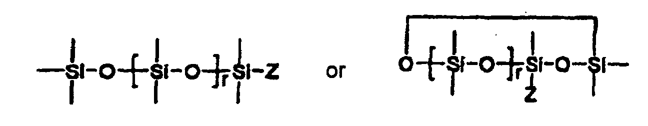 in which r is 0 to 6.

8. A compound of formula I according to claim 1:
5,5',5"-tris-(3-triethylsilyl)-propyloxy-2,2',2"-[1.3.5]-triazine-2,4,6-triyl-tris-phenol;
5,5'-di-[3-(1,1,1,3,5,5,-heptamethyl-trisilyl)-propyloxy]-5"-oxy-2,2',2"-[1.3.5]-triazine-2,4,6-triyl-tris-phenol;
5,5',5"-tris-[3-(1,1,1,3,3-pentamethyl-trisilyl)-propyloxy]-2,2',2"-[1.3.5]-triazine-2,4,6-triyl-tris-phenol;
5,5'-di-[tris-(3-trimethyl-siloxy-silyl)-propyloxy]-5"-oxy-2,2',2"-[1.3.5]-triazine-2,4,6-triyl-tris-phenol;
5,5',5"-tris-[3-triethoxysilyl-propyloxy]-2,2',2"-[1.3.5]-triazine-2,4,6-triyl-tris-phenol;
5,5'-di-[3-triethoxysilyl-propyloxy]-5"-oxy-2,2',2"-[1.3.5]-triazine-2,4,6-triyl-tris-phenol;
5,5',5"-tris-[2-(triethylsilyl)-2-methylene-ethoxy]-2,2',2"-[1.3.5]-triazine-2,4,6-triyl-tris-phenol;
5,5',5"-tris-[4-(1,1,1,3,3-pentamethyl-trisilyl)-butyloxy]-2,2',2"-[1.3.5]-triazine-2,4,6-triyl-tris-phenol;
5,5',5"-tris-[3-(tert.butyl-dimethyl-silyl)-propyloxy]-2,2',2"-[1.3.5]-triazine-2,4,6-triyl-tris-phenol;
5,5'-di-[3-(tert.butyl-dimethyl-silyl)-propyloxy]-5"-oxy-2,2',2"-[1.3.5]-triazine-2,4,6-triyl-tris-phenol;
5,5',5"-tris-[5-(1,1,1,1,3,3-pentamethyl-disiloxane-oxy)-pentyl-1-oxy]-2,2',2"-(1.3.5)-triazine-2,4,6-triyl-tris-phenol;
5,5',5"-tris-[4-(1,1,1,3,3-pentamethyl-disiloxane-oxy)-butyl-1-oxy]-2,2',2"-(1.3.5)-triazine-2,4,6-triyl-tris-phenol;
5,5',5"-tris-[6-(1,1,1,3,3-pentamethyl-disiloxane-oxy)-hexyl-1-oxy]-2,2',2"-(1.3.5)-triazine-2,4,6-triyl-tris-phenol.

9. A light screening agent, which contains at least one compound of formula I according to any one of claims 1 to 8 and optionally at least one additional UV filter.

## Revendications

1. Composé de la formule I, 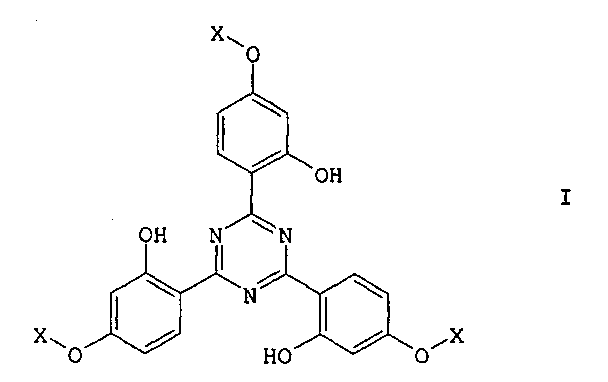 dans laquelle les X sont des résidus silicium S reliés à O par l'intermédiaire de groupes espaceurs Z ou deux X sont des résidus silicium S reliés à O par l'intermédiaire de groupes espaceurs Z et le troisième X est H ou un résidu silicium S relié à O,
le groupe espaceur Z étant un groupe alkyle saturé ou bien mono- ou pluri-insaturé de 3 à 12 atomes de carbone, et
le résidu silicium S contient de 1 à 8 atomes de Si et est constitué par des groupes alkylsilyle, alcoxysilyle trisubstitués ayant chacun 3 à 15 atomes de carbone ou par un oligosiloxane.

2. Composé de la formule I selon la revendication 1, dans lequel tous les X sont des résidus silicium S reliés à O par l'intermédiaire de groupes espaceurs Z.

3. Composé de la formule I selon la revendication 1 ou la revendication 2, dans lequel les groupes alkyle des groupes espaceurs Z sont reliés en position 2 ou 3 aux résidus silicium S.

4. Composé de la formule I selon la revendication 1 ou la revendication 2, dans lequel les groupes alkyle des groupes espaceurs Z sont reliés en position 4, 5 ou 6 aux résidus silicium S.

5. Composé de la formule I selon l'une des revendications 1 à 4, **caractérisé en ce que** les groupes espaceurs Z sont 3-propyl-, 2-propyl-, 3-propényl-, 2-propén-3-yl-, 2-propén-2-yl-, 2-méthyl-2-propyl, 2-méthyl-3-propényl-,
4-butyl-, 3-butyl-, 2-butyl-, 3-butényl-, 3-méthyl-2-butyl-, 2,4-pentadién-5-yl-, 4-pentyl-, 5-pentyl-, 5-hexyl-, 6-hexyl- ou 2-dodécén-12-yl- ; de préférence 3-propyl-, 4-butyl- ou 5-pentyl-.

6. Composé de la formule I selon l'une des revendications 1 à 5, **caractérisé en ce que** les résidus silicium S sont -Si-Me₃, -Si-Et₃, -Si-isopropényle₃-, -Si-tert-butyle₃, -Si-sec-butyle₃, -Si-Me₂-tert-butyle, -Si-Me₂-T-hexyle, -Si(OMe)₃, -Si(OEt)₃, -Si(OPh)₃ ou oligosiloxane ; de préférence -Si(OEt)₃ ou SiEt₃.

7. Composé de la formule 1 selon la revendication 6, **caractérisé en ce que** l'oligosiloxane est -SiMeₘ(OsiMe₃)ₙ où m = 0, 1 ou 2 ; n = 3, 2 ou 1 ; et m + n = 3 ; de préférence -SiMe₂OSiMe₃ ; ou un oligosiloxane des formules 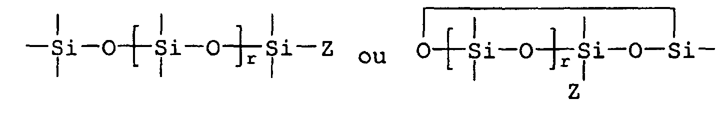 où r vaut de 0 à 6.

8. Composé de la formule 1 selon la revendication 1 :
5,5',5"-Tris-(3-triéthylsilyl)-propyloxy-2,2',2"-[1.3.5]-triazin-2,4,6-triyl-trisphénol ;
5,5'-Di-[3-(1,1,1,3,5,5,5-heptaméthyltrisilyl)-propyloxy]-5"-oxy-2,2',2"-[1.3.5]-triazin-2,4,6-triyltrisphénol ;
5,5',5"-Tris-[3-(1,1,1,3,3-pentaméthyltrisilyl)-propyloxy]-2,2',2"-[1.3.5]-triazin-2,4,6-triyltrisphénol ;
5,5'-Di-[tris-(3-triméthylsiloxysilyl)propyloxy]-5"-oxy-2,2',2"-[1.3.5]-triazin-2,4,6-triyl-trisphénol ;
5,5',5"-Tris-[3-triéthoxysilylpropyloxy]-2,2',2'-[1.3.5]-triazin-2,4,6-triyl-trisphénol ;
5,5'-Di-[3-triéthoxysilylpropyloxy]-5"-oxy-2,2',2"-[1.3.5]-triazin-2,4,6-triyl-trisphénol ;
5,5',5"-Tris-[2-(triéthylsilyl)-2-méthylèneéthoxy]-2,2',2"-[1.3.5]-triazin-2,4,6-triyl-trisphénol ;
5,5',5"-Tris-[4-(1,1,1,3,3-pentaméthyltrisilyl)-butyloxy]-2,2',2'-[1.3.5]-triazin-2,4,6-triyltrisphénol ;
5,5',5"-Tris-[3-(tert-butyldiméthylsilyl)propyloxy]-2,2',2'-[1.3.5]-triazin-2,4,6-triyl-trisphénol;
5,5'-Di-[3-(tert-butyldiméthylsilyl)-propyloxy]-5"-oxy-2,2',2"-[1.3.5]-triazin-2,4,6-triyl-trisphénol ;
5,5',5"-Tris-[5-(1,1,1,3,3-pentaméthyldisiloxanoxy)-pentyl-1-oxy]-2,2',2"-(1.3.5)-triazin-2,4,6-triyltrisphénol ;
5,5',5"-Tris-[4-(1,1,1,3,3-pentaméthyldisiloxanoxy)-butyl-1-oxy]-2,2',2"-(1.3.5)-triazin-2,4,6-triyltrisphénol ;
5,5',5"-Tris-[6-(1,1,1,3,3-pentaméthyldisiloxanoxy)-hexyl-1-oxy]-2,2',2'-(1.3.5)-triazin-2,4,6-triyltrisphénol.

9. Agent photoprotecteur, **caractérisé en ce qu'**il contient au moins un composé de la formule 1 selon l'une au moins des revendications 1 et 8 et le cas échéant au moins un autre filtre anti-UV.
